(19) Europäisches Patentamt
European Patent Office
Office européen des brevets

(11) Numéro de publication: 0 216 714 B1

(12) **FASCICULE DE BREVET EUROPEEN**

(45) Date de publication de fascicule du brevet: **13.05.92**

(51) Int. Cl.[5]: **C07C 37/62**, C07C 39/32

(21) Numéro de dépôt: **86420221.3**

(22) Date de dépôt: **03.09.86**

(54) **Procédé de chloration sélective de composés phénoliques.**

(30) Priorité: **19.09.85 FR 8514093**

(43) Date de publication de la demande:
**01.04.87 Bulletin 87/14**

(45) Mention de la délivrance du brevet:
**13.05.92 Bulletin 92/20**

(84) Etats contractants désignés:
**AT BE CH DE FR GB IT LI LU NL SE**

(56) Documents cités:
**DE-A- 3 318 791**
**FR-A- 920 514**
**GB-A- 2 006 189**

**CHEMICAL ABSTRACTS, vol. 93, no. 25, 22 décembre 1980, page 814, résumé no. 239023s, Columbus, Ohio, US; & JP-A-80 40 613**

(73) Titulaire: **RHONE-POULENC CHIMIE**
**25, quai Paul Doumer**
**F-92408 Courbevoie Cédex(FR)**

(72) Inventeur: **Leblanc, Jean-Claude**
**10, boulevard Joffre**
**F-38000 Grenoble(FR)**
Inventeur: **Ratton, Serge**
**Hameau de Belmont Vaulx-Milieu**
**F-38090 Villefontaine(FR)**

(74) Mandataire: **Vignally, Noel et al**
**RHONE-POULENC INTERSERVICES Service Brevets Chimie Centre de Recherches de Saint-Fons B.P. 62**
**F-69192 Saint-Fons Cedex(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 216 714 B1

Rank Xerox (UK) Business Services
(3.08/2.19/2.0)

## Description

La présente invention concerne la chloration sélective par le chlore gazeux de composés phénoliques, en position ortho par rapport à la fonction hydroxyle.

Dans la préparation des chlorophénols par chloration du phénol, on obtient aux divers stades de la réaction des isomères, ce qui conduit finalement à de nombreux composés, qu'il est ensuite nécessaire de séparer par des opérations coûteuses et délicates. D'autre part les rapports des différents isomères obtenus ne correspondent pas forcément à ce qui serait économiquement intéressant, compte-tenu des débouchés existants.

Il est donc apparu souhaitable de disposer de procédés de chloration sélective, notamment de chloration sélective en position ortho ou en position para par rapport à la fonction hydroxyle du composé phénolique.

Ainsi la demande de brevet allemand No. 3 318 791 décrit un procédé sélectif de chloration du phénol en chloro-2 phénol, dans un solvant apolaire perchloré et en présence d'une amine à chaîne ramifiée.

Il est également connu selon le résumé du Chemical Abstracts 93:239023s (1980) un procédé de préparation de dichloro-2,6 alkyl-4 phénols par chloration d'alkyl-4 phénols, dans un solvant organique et en présence d'un catalyseur de type amine.

Cependant l'utilisation d'un solvant dans un procédé industriel de chloration peut, dans certains cas, présenter des inconvénients, tels que par exemple la nécessité d'un volume de réacteur plus important et une séparation plus difficile des produits de la réaction.

La présente invention a pour objet un procédé de chloration sélective de composés phénoliques par le chlore gazeux en milieu fondu et en présence d'une amine.

Plus précisément il s'agit d'un procédé de chloration sélective en position ortho par rapport à la fonction hydroxyle des composés phénoliques de formule générale (I) :

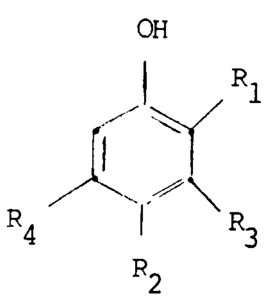

(I)

dans laquelle :

- $R_2$ représente :
  - . un atome d'halogène ;
  - . un groupement alkyle ayant 1 à 4 atomes de carbone ;
  - . un groupement alkoxy ayant 1 à 4 atomes de carbone ;
  - . un groupement acyloxy ayant 2 à 4 atomes de carbone ;
  - . un groupement acyle ayant 2 à 4 atomes de carbone ;
  - . un groupement acide carboxylique ;
  - . un groupement ester $-COOR_5$, $R_5$ représentant un radical alkyle linéaire ou ramifié ayant de 1 à 4 atomes de carbone ;
  - . un groupement nitrile ;
  - . un groupement OH ;
  - . un groupement -CHO ;
- $R_1$ représente un atome d'hydrogène ou possède l'une des significations indiquées pour $R_2$ ;
- $R_3$ et $R_4$, identiques ou différents, représentent un atome d' hydrogène ou un atome ou groupement tel que ceux représentés par $R_2$ ;

par le chlore gazeux, caractérisé en ce que l'on opère en milieu fondu et en présence d'une quantité efficace d'une amine primaire, secondaire ou tertiaire.

Par amine on entend dans le présent texte tout composé, liquide ou solide dans les conditions opératoires du procédé, comportant une ou plusieurs fonctions amine.

Un tel composé peut également comporter une ou plusieurs autres fonctions chimiques, telles que par exemple la fonction hydroxyle, la fonction acide carboxylique, la fonction ester d'acide carboxylique, la fonction amide ou la fonction imine.

Il est bien évident que les amines utilisées peuvent être introduites également sous la forme de leurs chlorhydrates respectifs.

Les amines utilisées comme catalyseur dans le présent procédé sont plus particulièrement les amines de formule générale (II)

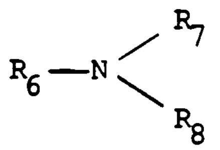

(II)

dans laquelle :

- $R_6$, $R_7$ et $R_8$, identiques ou différents, représentent :
  . un radical alkyle linéaire de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther -O- ou fonctions hydroxyle, amine, acide carboxylique, ester d'acide carboxylique, amide ou imine ;
  . un radical phényle, un radical cyclohexyle, un radical cycloheptyle ou un radical cyclopentyle ;
  . un radical phénylalkyle, cyclohexylalkyle, cycloheptylalkyle ou cyclopentylalkyle dont la partie alkyle comporte 1 à 4 atomes de carbone :
- un ou deux des symboles $R_6$, $R_7$ et $R_8$ peuvent représenter un atome d'hydrogène ;
- $R_6$ peut représenter un groupement $NH_2$ ;
- $R_7$ et $R_8$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone,
- $R_7$ et $R_8$ ou $R_6$, $R_7$ et $R_8$ forment ensemble, avec l'atome d'azote et avec un ou plusieurs atomes d'azote et/ou d'oxygène, un hétérocycle, saturé ou insaturé, éventuellement substitué par un ou plusieurs groupements alkyles ayant 1 à 4 atomes de carbone ;
- $R_6$, $R_7$ et $R_8$ forment ensemble et avec l'atome d'azote un hétérocycle insaturé éventuellement substitué par un ou deux groupements méthyle ou éthyle ;
- $R_7$ et $R_8$ ou $R_6$, $R_7$ et $R_8$ forment ensemble, avec l'atome d'azote et éventuellement avec un ou plusieurs atomes d'azote et/ou d'oxygène, un composé polycyclique, saturé ou insaturé, éventuellement substitué par un ou plusieurs groupements alkyles ayant 1 à 4 atomes de carbone.

A titre d'exemples illustratifs, on peut citer comme amines de formule (II) :

- des amines primaires telles que la n-propylamine, l'isopropylamine, l'isobutylamine, la n-butylamine, la tertiobutylamine, la n-pentylamine, la méthyl-2 butylamine, la méthyl-3 butylamine, la n-hexylamine, l'éthyl-2 hexylamine, l'aniline, la laurylamine, la cyclohexylamine, la cyclopentylamine, la benzylamine, la guanidine, l'acétamidine, l'ester éthylique de la glycine, l'éthanolamine, l'éthylènediamine, l'hexaméthylènediamine, la N-aminoéthylpyrrolidine, la pyrazoline, la lysine, la N-aminomorpholine et la N-aminopipéridine ;
- des amines secondaires telles que la dibutylamine, la dipropylamine, la méthylpropylamine, la méthylbutylamine, la méthylisobutylamine, la méthyltertiobutylamine, la méthylbenzylamine, la pipéridine, la diisopropylamine, la diisobutylamine, la ditertiobutylamine, la méthyl-1 cyclopentylamine, la méthyl-1 cyclohexylamine, la dicyclohexylamine, la morpholine, l'imidazole, la pyrrolidine, l'imidazolidine, la pipérazine, l'indole ;
- des amines tertiaires telles que la triéthylamine, la tributylamine, la pyridine, la tris(dioxa-3,6 heptyl)-amine, le diaza-1,8 bicyclo[5,4,0]undécène-7.

On peut utiliser également des composés aminés comme l'hydrazine ou ses dérivés, notamment les dérivés obtenus par substitution d'un ou de deux atomes d'hydrogène par des radicaux alkyles, aryles, cycloaliphatiques ou hétérocycliques.

La quantité d'amine utilisée dans le procédé peut varier dans de très larges limites.

Habituellement elle représente, en poids par rapport au poids du mélange réactionnel de 0,005 % à 5 %. Préférentiellement on mettra en oeuvre de 0,01 % à 2,0 % en poids d'amine par rapport au mélange réactionnel, afin d'avoir une efficacité suffisante, tout en n'ayant pas une quantité excessive d'amine qui n'apporte pas d'avantage supplémentaire.

Parmi les amines de formule générale (II), on préfère dans le cadre du présent procédé, les amines primaires ou secondaires de formule générale (III) :

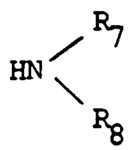

(III)

dans laquelle :

- $R_7$ ou $R_8$ représente un atome d'hydrogène ;
- $R_7$ et $R_8$, identiques ou différents, représentent :
  . un radical alkyle linéaire ayant 1 à 10 atomes de carbone ;
  . un radical alkyle secondaire ayant 3 à 10 atomes de carbone ;
  . un radical alkyle tertiaire ayant 4 à 10 atomes de carbone ;
  . un radical cyclohexyle ou cyclopentyle ;
  . un radical phényle ;
  . un radical benzyle ou phénéthyle ;
- $R_7$ et $R_8$ forment ensemble, avec l'atome d'azote et avec un autre atome d'azote et/ou d'oxygène, un hétérocycle saturé ou comportant une ou plusieurs insaturations ;
- $R_7$ et/ou $R_8$ comportent une ou plusieurs fonctions amine, hydroxyle ou ester d'acide carboxylique.

A titre d'exemples d'amines primaires de formule générale (III), on peut citer la n-propylamine, l'isopropylamine, la n-butylamine, l'isobutylamine, la tertiobutylamine, la n-pentylamine, la méthyl-2 butylamine, la méthyl-3 butylamine, la n-hexylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine et l'éthanolamine.

En ce qui concerne plus particulièrement les amines secondaires de formule générale (III), on préfère encore plus spécialement celles pour lesquelles au moins un des symboles $R_7$ et $R_8$ et de préférence les deux symboles $R_7$ et $R_8$ représentent :

. un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;
. un radical cyclohexyle ou cyclopentyle ;

ou celles pour lesquelles $R_7$ et $R_8$ forment avec l'atome d'azote un hétérocycle comportant éventuellement un autre atome d'azote ou un atome d'oxygène.

A titre d'exemples de telles amines secondaires, on peut citer la diisopropylamine, la diisobutylamine, la dicyclohexylamine, la morpholine, l'imidazole.

Les composés phénoliques de formule (I), pour lesquels le présent procédé offre le plus d'intérêt, sont ceux dans la formule desquels :

- $R_2$ représente :
  . un atome de chlore, un atome de fluor ou un atome de brome ;
  . un groupement méthoxy ou éthoxy ;
  . un groupement aldéhyde ;
  . un groupement OH ;
  . un groupement CN ;
  . un groupement acétoxy ;
  . un groupement ester $-COOR_5$, $R_5$ représentant un groupement méthyle ou éthyle ;
  . un groupement acyle ayant 2 à 4 atomes de carbone ;
- $R_1$ représente un atome d'hydrogène, un groupement méthyle, éthyle, propyle, isopropyle ou tertiobutyle ou a les significations indiquées pour $R_2$ ;
- $R_3$ et $R_4$ représentent :
  . l'un un atome d'hydrogène ;
  . l'autre un atome d'hydrogène ou un groupement tel que ceux représentés par $R_2$.

A titre d'exemples de composés phénoliques de formule (I), on peut citer notamment : le chloro-4 phénol, le dichloro-2,4 phénol, le dichloro-3,4 phénol, le chloro-2 fluoro-4 phénol, le chloro-4 fluoro-2 phénol, le bromo-2 chloro-4 phénol, le bromo-4 chloro-2 phénol, le chloro-2 méthoxy-4 phénol, le chloro-4 méthoxy-2 phénol, le chloro-4 méthyl-2 phénol, le méthoxy-4 phénol, l'éthoxy-4 phénol, l'hydroxy-4 acétophénone, l'hydroxy-4 benzonitrile, le chloro-3 hydroxy-4 benzaldéhyde, le chloro-5 hydroxy-2 benzaldéhyde.

Le présent procédé possède plusieurs avantages.

Un de ces avantages consiste dans sa sélectivité intramoléculaire : lorsqu'il est appliqué à un composé phénolique de formule (I) dans laquelle au moins une des positions en méta de la fonction hydroxyle est libre, pratiquement seules les positions en ortho de l'OH sont chlorées, alors qu'en l'absence d'amine, il se forme toujours une quantité non négligeable du chloro-3 phénol ou du chloro-5 phénol correspondant, qu'il est ensuite très difficile de séparer. En outre ces composés peuvent être très gênants pour certaines applications.

Un autre avantage très intéressant concerne la sélectivité intermoléculaire du procédé : si le procédé est mis en oeuvre avec un mélange comportant un composé phénolique de formule (I) et un ou plusieurs autres composés phénoliques [tels que par exemple des isomères de position dudit composé phénolique de formule (I) ne] répondant pas à la formule (I), c'est en grande majorité le composé phénolique de formule (I) qui est chloré, tandis que le ou les autres composés phénoliques ne sont que peu transformés.

Ainsi par exemple le procédé de l'invention peut avantageusement être appliqué à un mélange de dichloro-2,4 phénol et de dichloro-2,6 phénol ; on obtient du trichloro-2,4,6 phénol à partir du dichloro-2,4 phénol, tandis que le dichloro-2,6 phénol, qui n'est que peu transformé, peut être relativement facilement séparé du mélange réactionnel final.

On peut également mettre en oeuvre un mélange brut industriel contenant du dichloro-2,4 phénol, du dichloro-2,6 phénol et du trichloro-2,4,6 phénol et obtenir ainsi comme précédemment du trichloro-2,4,6 phénol et du dichloro-2,6 phénol, qui peuvent être séparés par distillation fractionnée.

Enfin un autre intérêt du présent procédé tient à ce que la réaction du chlore gazeux et du composé phénolique de formule (I) est pratiquement complète, alors qu'en l'absence d'amine une partie importante du chlore ne réagit pas. Les problèmes de recyclage de l'excès de chlore ou de traitement des effluents gazeux deviennent ainsi moins difficiles à résoudre.

La quantité de chlore utilisée dans le procédé de l'invention est par conséquent essentiellement fonction du taux de transformation souhaité du composé phénolique (I).

Le chlore peut être utilisé seul ou être dilué par un gaz inerte, tel que l'azote par exemple. La présence d'un gaz inerte permet, si nécessaire, d'augmenter le débit gazeux sans augmenter la quantité de chlore introduite en un temps donné.

La température à laquelle est mis en oeuvre le procédé est généralement inférieure ou égale à 150 ° C. D'autre part la limite inférieure de la zone de température est conditionnée par le point de fusion du composé phénolique (I) utilisé ou du mélange de composés phénoliques.

Généralement cette température est comprise entre le point de fusion du mélange réactionnel et 120 ° C, mais sans que ces chiffres aient une valeur critique.

Les exemples qui suivent ont pour but d'illustrer le procédé l'invention.

## EXEMPLE 1

Dans un réacteur en verre de 100 cm3, équipé d'un agitateur, d'une tubulure permettant l'arrivée du chlore gazeux, d'un thermomètre et surmonté d'un réfrigérant, on introduit les charges suivantes :

- dichloro-2,4 phénol : 21,19 g (130 millimoles)
- dichloro-2,6 phénol : 21,19 g (130 millimoles)
- diisopropylamine : 0,042 g (0,42 millimole soit 0,1 % en poids dans le milieu réactionnel).

On porte la température à 70 ° C sous agitation de façon à ce que le mélange soit à l'état liquide et on commence alors à introduire le chlore gazeux avec un débit de 5 litres/heure.

La durée de la chloration effectuée à 70 ° C est de 38 minutes, ce qui correspond à 3,15 litres de chlore introduit (140,6 millimoles). L'essai est donc réalisé avec un excés de 8 % de chlore par rapport au dichloro-2,4 phénol.

En fin de réaction, on purge l'ensemble de l'installation par un courant d'azote. On obtient une masse réactionnelle de 47,21 g, que l'on analyse par chromatographie en phase vapeur en présence d'un étalon interne.

On obtient les résultats suivants :

- taux de transformation (TT) du dichloro-2,4 phénol : 99,6 %
- taux de transformation (TT) du dichloro-2,6 phénol : 6,6 %

- rendement (RT) en trichloro-2,4,6 phénol par rapport aux dichlorophénols transformés : 100 %.

$$- \text{rapport } \frac{\text{TT du dichloro-2,4 phénol}}{\text{TT du dichloro-2,6 phénol}} = 15{,}1$$

EXEMPLE 2

Dans un réacteur en verre de 200 cm3, équipé comme indiqué dans l'exemple 1, on introduit les charges suivantes :

- dichloro-2,4 phénol : 109,15 g (669,6 millimoles)
- dichloro-2,6 phénol : 24,78 g (152 millimoles)
- diisopropylamine : 0,13 g (1,29 millimole soit 0,1 % en poids dans le milieu réactionnel).

On introduit à 70 ° C, sous agitation, 15 litres de chlore (669,6 millimoles) avec un débit de 5 l/h, ce qui correspond à la quantité stoechiométrique par rapport au dichloro-2,4 phénol.

Par dosage chromatographique à la fin de la chloration on obtient les résultats suivants :

- TT du dichloro-2,4 phénol : 99,95 %
- TT du dichloro-2,6 phénol : 8,1 %
- RT en trichloro-2,4,6 phénol par rapport aux dichlorophénols transformés : 99,1 %.

$$- \text{rapport } \frac{\text{TT du dichloro-2,4 phénol}}{\text{TT du dichloro-2,6 phénol}} = 12{,}3$$

EXEMPLES 3 à 6

On opère comme dans l'exemple 1 à 70 ° C avec un mélange de dichloro-2,4 phénol et de dichloro-2,6 phénol 50/50. Le chlore gazeux est introduit avec un débit de 5 l/h et la quantité introduite est telle que l'on a un rapport molaire chlore/dichloro-2,4 phénol de 0,95.

On introduit les charges suivantes :

- dichloro-2,4 phénol : 21,19 g (130 millimoles)
- dichloro-2,6 phénol : 21,19 g (130 millimoles)
- diisopropylamine : quantité variable (voir tableau I)
- chlore : 2,765 litres (123 millimoles).

Le tableau (I) rassemble les caractéristiques des différents exemples ainsi que les résultats obtenus.

Tableau (I)

| EXAMPLES | Diisopropylamine poids en g % en poids/DCP | TT du DCP-2,4 (*) | TT du DCP-2,6 (**) | RT en TCP-2,4,6 (***) | TT du DCP-2,4/TT du DCP-2,6 |
|---|---|---|---|---|---|
| Essai témoin A | 0 | 47,1 % | 38,6 % | 97,2 % | 1,2 |
| Exemple 3 | 0,01 g<br>0,0235 % | 73,8 % | 10,0 % | 100 % | 7,4 |
| Exemple 4 | 0,042 g<br>0,10 % | 87,0 % | 4,7 % | 100 % | 18,5 |
| Exemple 5 | 0,094 g<br>0,225 % | 82,2 % | 3,8 % | 100 % | 21,6 |
| Exemple 6 | 0,84 g<br>2,0 % | 83,5 % | 7,2 % | 100 % | 11,6 |

(*) = TT du dichloro-2,4 phénol
(**) = TT du dichloro-2,6 phénol
(***) = RT en trichloro-2,4,6 phénol.

Ces essais montrent l'influence favorable de l'amine utilisée pour des rapports pondéraux amine/mélange réactionnel très variés.

EXEMPLES 7 à 11

Ces essais sont effectués avec différentes amines utilisées à même teneur pondérale (0,02 % par rapport aux dichlorophénols).

On opère comme dans la série d'exemples 3 à 6 avec les charges suivantes :

- dichloro-2,4 phénol : 21,19 g (130 millimoles)
- dichloro-2,6 phénol : 21,19 g (130 millimoles)
- amine : 0,02 % en poids par rapport aux dichlorophénols
- chlore : 2,766 litres (123 millimoles).

Le tableau (II) rassemble les caractéristiques des différents exemples ainsi que les résultats obtenus (l'exemple 3 est repris à titre de comparaison).

Tableau (II)

| EXEMPLES | amine utilisée | TT du DCP-2,4 | TT du DCP-2,6 | RT en TCP-2,4,6 | TT du DCP-2,4/TT du DCP-2,6 |
|---|---|---|---|---|---|
| Exemple 3 | diisopropylamine | 73,8 % | 10,0 % | 100 % | 7,4 |
| Exemple 7 | méthyl-n-butylamine | 69,9 % | 15,8 % | 100 % | 4,4 |
| Exemple 8 | dicylohexylamine | 76,9 % | 14,2 % | 99,0 % | 5,4 |
| Exemple 9 | tributylamine | 53,0 % | 33,1 % | 98,6 % | 1,6 |
| Exemple 10 | tris(dioxa-3,6 heptyl)amine | 60,45 % | 25,1 % | 100 % | 2,4 |
| Exemple 11 | pyridine | 60,4 % | 29,2 % | 97,6 % | 2,1 |

On note, à concentration pondérale égale, un effet plus important des amines secondaires par rapport aux amines tertiaires.

EXEMPLES 12 à 13

Ces essais sont effectués avec trois amines différentes, utilisées à même teneur molaire (0,15 % à 0,18 % en mole par rapport aux dichlorophénols).

On opère comme dans la série d'exemples 3 à 6 avec les charges suivantes :

- dichloro-2,4 phénol : 21,19 g (130 millimoles)
- dichloro-2,6 phénol : 21,19 g (130 millimoles)
- amine : voir tableau (III)
- chlore : 2,766 litres (123 millimoles).

Le tableau (II) rassemble les caractéristiques des différents exemples ainsi que les résultats obtenus (l'exemple 4 est repris à titre de comparaison).

Tableau (III)

| EXEMPLES | amine % en mole/DCP (% en poids/DCP) | TT du DCP-2,4 | TT du DCP-2,6 | RT en TCP-2,4,6 | TT du DCP-2,4/TT du DCP-2,6 |
|---|---|---|---|---|---|
| Exemple 4 | diisopropylamine 0,18 % en mole (0,1 % en poids) | 87,0 % | 4,7 % | 100 % | 18,5 |
| Exemple 12 | dicyclohexylamine 0,18 % en mole (0,2 % en poids) | 89,0 % | 5,15 % | 99,4 % | 17,3 |
| Exemple 13 | tris(dioxa-3,6 heptyl)amine 0,155 % en mole (0,3 % en poids) | 75,8 % | 17,25 % | 99,3 % | 4,4 |

EXEMPLE 14

On opère comme dans la série d'exemples 3 à 6 avec les charges suivantes :

- dichloro-2,4 phénol : 21,19 g (130 millimoles)
- dichloro-2,6 phénol : 21,19 g (130 millimoles)
- diisopropylamine : 0,042 g (0,42 millimole soit 0,1 % en poids par rapport aux dichlorophénols)
- chlore : 2,766 litres (123 millimoles).

Le tableau (IV) rassemble les caractéristiques de l'exemple ainsi que les résultats obtenus (l'exemple 4 est repris à titre de comparaison).

Tableau (IV)

| EXEMPLES | Température | TT du DCP-2,4 | TT du DCP-2,6 | RT en TCP-2,4,6 | TT du DCP-2,4/TT du DCP-2,6 |
|---|---|---|---|---|---|
| Exemple 4 | 70 °C | 87,0 % | 4,7 % | 100 % | 18,5 |
| Exemple 14 | 100 °C | 88,3 % | 4,55 % | 99,9 % | 19,4 |

EXEMPLE 15

Dans un réacteur en verre de 200 cm3, équipé d'un agitateur, d'une tubulure permettant l'arrivée du chlore gazeux, d'un thermomètre et surmonté d'un réfrigérant, on introduit les charges suivantes :

- dichloro-2,4 phénol : 100 g (613,5 millimoles)
- diisopropylamine : 0,10 g (1 millimole soit 0,1 % en poids dans le milieu réactionnel).

On porte la température à 70 °C sous agitation de façon à ce que le mélange soit à l'état liquide et on commence alors à introduire le chlore gazeux avec un débit de 5 litres/heure.

La durée de la chloration effectuée à 70 °C est de 3 heures, ce qui correspond à 670 millimoles de chlore introduit. L'essai est donc réalisé avec un excès de 10 % de chlore par rapport au dichloro-2,4 phénol.

En fin de réaction, on purge l'ensemble de l'installation par un courant d'azote. On obtient une masse réactionnelle que l'on analyse par chromatographie en phase vapeur en présence d'un étalon interne.

On obtient les résultats suivants :

- dichloro-2,4 phénol restant : 0,09 g, soit un TT de 99,9 %

- trichloro-2,4,6 phénol obtenu : 119,69 g, soit un RT par rapport au dichloro-2,4 phénol transformé de 98,9 %
- teneur en trichloro-2,4,5 phénol : inférieure à 0,001 % en poids dans la masse réactionnelle (estimée à 0,0007 %).

EXEMPLE 16

On répète l'exemple 15 avec une concentration en diisopropylamine vingt fois plus élevée.

On charge donc :

- dichloro-2,4 phénol : 100 g (613,5 millimoles)
- diisopropylamine : 2,0 g (20 millimoles soit 2 % en poids dans le milieu réactionnel).

On obtient les résultats suivants :

- dichloro-2,4 phénol restant : 0,08 g, soit un TT de 99,9 %
- trichloro-2,4,6 phénol obtenu : 121,05 g, soit un RT par rapport au dichloro-2,4 phénol transformé de 100 %
- teneur en trichloro-2,4,5 phénol : inférieure à 0,001 % en poids dans la masse réactionnelle (estimée à 0,0004 %).

EXEMPLES 17 et 18

On opère comme dans la série d'exemples 3 à 6 avec les charges suivantes :

- dichloro-2,4 phénol : 21,19 g (130 millimoles)
- dichloro-2,6 phénol : 21,19 g (130 millimoles)
- amine (voir tableau) : 0,042 g (0,1 % en poids par rapport aux dichlorophénols)
- chlore (débit 5 l/h) : exemple 17 = 2,750 litres (123 millimoles)
  exemple 18 = 2,910 litres (130 millimoles).

Le tableau (V) rassemble les caractéristiques des deux exemples ainsi que les résultats obtenus.

Tableau (V)

| EXEMPLES | amine utilisée % en poids/DCP | TT du DCP-2,4 | TT du DCP-2,6 | RT en TCP-2,4,6 | TT du DCP-2,4/TT du DCP-2,6 |
|---|---|---|---|---|---|
| Exemple 17 | isopropylamine 0,1 % en poids | 87,45 % | 6,55 % | 98,7 % | 13,3 |
| Exemple 18 | n-propylamine 0,1 % en poids | 94,9 % | 6,8 % | 98,5 % | 13,9 |

ESSAI TEMOIN B

On répète l'exemple 15 sans diisopropylamine.

Afin d'avoir un taux de transformation suffisant du dichloro-2,4 phénol, il est nécessaire d'introduire un excès de chlore de 60 % par rapport à la quantité stoechiométrique.

On obtient les résultats suivants :

- dichloro-2,4 phénol restant : 0,94 g, soit un TT de 99,05 %
- trichloro-2,4,6 phénol obtenu : 120,89 g, soit un RT par rapport au dichloro-2,4 phénol transformé de 100 %
- teneur en trichloro-2,4,5 phénol : 0,003 % en poids dans la masse réactionnelle.

ESSAI TEMOIN C

On répète l'essai témoin B, mais on arrête l'arrivée du chlore lorsque le rapport molaire chlore engagé/dichloro-2,4 phénol chargé est égal à 1,1 (ce qui représente un excès de chlore de 10 % par rapport à la quantité stoechiométrique).

On obtient les résultats suivants :

- TT du dichloro-2,4 phénol : 91,0 %
- RT en trichloro-2,4,6 phénol : 100 %
- teneur en trichloro-2,4,5 phénol : 0,0087 % en poids dans la masse réactionnelle.

EXEMPLES 19 à 31

Ces essais sont effectués avec différentes amines utilisées à même teneur pondérale (0,1 % par rapport aux dichlorophénols).

On opère comme dans la série d'exemples 3 à 6 avec les charges suivantes :

- dichloro-2,4 phénol : 21,19 g (130 millimoles)
- dichloro-2,6 phénol : 21,19 g (130 millimoles)
- amine : 0,1 % en poids par rapport aux dichlorophénols
- chlore : 2,766 litres (123 millimoles).

Le tableau (VI) rassemble les caractéristiques des différents exemples ainsi que les résultats obtenus.

Tableau (VI)

| EXEMPLES | amine utilisée | TT du DCP-2,4 | TT du DCP-2,6 | RT en TCP-2,4,6 | TT du DCP-2,4/TT du DCP-2,6 |
|---|---|---|---|---|---|
| Exemple 19 | morpholine | 83,8 % | 7,4 % | 98 % | 11,3 |
| Exemple 20 | chlorhydrate de guanidine | 77,3 % | 15,7 % | 98,5 % | 4,9 |
| Exemple 21 | chlorhydrate d'acétamidine | 81,0 % | 10,2 % | 100 % | 7,9 |
| Exemple 22 | imidazole | 78,2 % | 13,4 % | 99 % | 5,8 |
| Exemple 23 | DBU (*) | 71,9 % | 18,9 % | 98 % | 3,8 |
| Exemple 24 | ester éthylique de la glycine | 79,2 % | 12,1 % | 99 % | 6,5 |
| Exemple 25 | N-aminomorpholine | 74,3 % | 12,7 % | 97,2 % | 5,8 |
| Exemple 26 | N-aminopipéridine | 68,8 % | 19,4 % | 98 % | 3,5 |
| Exemple 27 | N-aminoéthylpyrrolidine | 81,1 % | 10,3 % | 97,8 % | 7,9 |
| Exemple 28 | éthanolamine | 84,6 % | 8,6 % | 98 % | 9,8 |
| Exemple 29 | benzylamine | 82,1 % | 8,3 % | 98 % | 9,9 |
| Exemple 30 | éthylènediamine | 61,3 % | 27,8 % | 98 % | 2,3 |
| Exemple 31 | L-lysine | 63,7 % | 25,8 % | 96 % | 2,5 |

(*) DBU = diaza-1,8 bicyclo[5,4,0]undécène-7.

## Revendications

**1.** Procédé de chloration sélective en position ortho par rapport à la fonction hydroxyle des composés phénoliques de formule générale (I) :

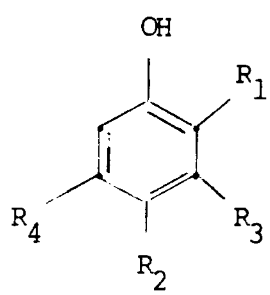

(I)

dans laquelle :

- $R_2$ représente :
  . un atome d'halogène ;

. un groupement alkyle ayant 1 à 4 atomes de carbone ;
. un groupement alkoxy ayant 1 à 4 atomes de carbone ;
. un groupement acyloxy ayant 2 à 4 atomes de carbone ;
. un groupement acyle ayant 2 à 4 atomes de carbone ;
. un groupement acide carboxylique ;
. un groupement ester $-COOR_5$, $R_5$ représentant un radical alkyle linéaire ou ramifié ayant de 1 à 4 atomes de carbone ;
. un groupement nitrile ;
. un groupement OH ;
. un groupement -CHO ;

- $R_1$ représente un atome d'hydrogène ou possède l'une des significations indiquées pour $R_2$ ;
- $R_3$ et $R_4$, identiques ou différents, représentent un atome d'hydrogène ou un atome ou groupement tel que ceux représentés par $R_2$ ;

par le chlore gazeux, caractérisé en ce que l'on opère en milieu fondu et en présence d'une quantité efficace d'une amine primaire, secondaire ou tertiaire.

**2.** Procédé selon la revendication 1, caractérisé en ce que les amines utilisées sont plus particulièrement les amines de formule générale (II)

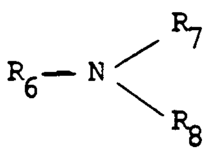

(II)

dans laquelle :

- $R_6$, $R_7$ et $R_8$, identiques ou différents, représentent :
  . un radical alkyl linéaire de 1 à 12 atomes de carbone, un radical alkyle secondaire ayant 3 à 12 atomes de carbone, ou un radical alkyle tertiaire ayant 4 à 12 atomes de carbone, ces radicaux alkyles pouvant comporter une ou deux fonctions éther -O- ou fonctions hydroxyle, amine, acide carboxylique, ester d'acide carboxylique, amide ou imine ;
  . un radical phényle, un radical cyclohexyle, un radical cycloheptyle ou un radical cyclopentyle ;
  . un radical phénylalkyle, cyclohexylalkyle, cycloheptylalkyle ou cyclopentylalkyle dont la partie alkyle comporte 1 à 4 atomes de carbone :
- un ou deux des symboles $R_6$, $R_7$ et $R_8$ peuvent représenter un atome d'hydrogène ;
- $R_6$ peut représenter un groupement $NH_2$ ;
- $R_7$ et $R_8$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone,
- $R_7$ et $R_8$ ou $R_6$, $R_7$ et $R_8$ forment ensemble, avec l'atome d'azote et avec un ou plusieurs atomes d'azote et/ou d'oxygène, un hétérocycle, saturé ou insaturé, éventuellement substitué par un ou plusieurs groupements alkyles ayant 1 à 4 atomes de carbone ;
- $R_6$, $R_7$ et $R_8$ forment ensemble et avec l'atome d'azote un hétérocycle insaturé éventuellement substitué par un ou deux groupements méthyle ou éthyle ;
- $R_7$ et $R_8$ ou $R_6$, $R_7$ et $R_8$ forment ensemble, avec l'atome d'azote et éventuellement avec un ou plusieurs atomes d'azote et/ou d'oxygène, un composé polycyclique, saturé ou insaturé, éventuellement substitué par un ou plusieurs groupements alkyles ayant 1 à 4 atomes de carbone.

**3.** Procédé selon l'une des revendications 1 et 2, caractérisé en ce que la quantité d'amine utilisée représente en poids par rapport au poids du mélange réactionnel de 0,005 % à 5 %, et de préférence de 0,01 % à 2,0 %.

4. Procédé selon l'une des revendications 1 à 3, caractérisé en ce que l'on utilise des amines primaires ou secondaires de formule générale (III) :

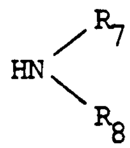

(III)

dans laquelle :

- $R_7$ ou $R_8$ représente un atome d'hydrogène ;
- $R_7$ et $R_8$, identiques ou différents, représentent :
  . un radical alkyle linéaire ayant 1 à 10 atomes de carbone ;
  . un radical alkyle secondaire ayant 3 à 10 atomes de carbone ;
  . un radical alkyle tertiaire ayant 4 à 10 atomes de carbone ;
  . un radical cyclohexyle ou cyclopentyle ;
  . un radical phényle ;
  . un radical benzyle ou phénéthyle.
- $R_7$ et $R_8$ forment ensemble, avec l'atome d'azote et avec un autre atome d'azote et/ou d'oxygène, un hétérocycle saturé ou comportant une ou plusieurs insaturations ;
- $R_7$ et/ou $R_8$ comportent une ou plusieurs fonctions amine, hydroxyle ou ester d'acide carboxylique.

5. Procédé selon l'une des revendications 1 à 4, caractérisé en ce que l'on utilise des amines secondaires de formule générale (III) :

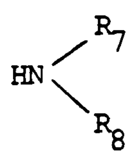

(III)

dans laquelle au moins un des symboles $R_7$ et $R_8$ et de préférence les deux symboles $R_7$ et $R_8$ représentent :

. un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;
. un radical cyclohexyle ou cyclopentyle.

ou des amines secondaires de formule générale (III) dans laquelle $R_7$ et $R_8$ forment avec l'atome d'azote un hétérocycle comportant éventuellement un autre atome d'azote ou un atome d'oxygène.

6. Procédé selon l'une des revendications 1 à 5, caractérisé en ce que l'on utilise comme amine la diisopropylamine, la diisobutylamine, la dicyclohexylamine, la morpholine ou l'imidazole.

7. Procédé selon l'une des revendications 1 à 6, caractérisé en ce que l'on utilise comme amine la n-propylamine, l'isopropylamine, la n-butylamine, l'isobutylamine, la tertiobutylamine, la n-pentylamine, la méthyl-2 butylamine, la méthyl-3 butylamine, la n-hexylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine, la N-aminomorpholine, la N-aminopipéridine, la N-aminoéthylpyrrolidine ou l'éthanolamine.

8. Procédé selon l'une des revendications 1 à 7, caractérisé en ce qu'il est appliqué aux composés phénoliques de formule générale (I) dans laquelle :

- $R_2$ représente :
  . un atome de chlore, un atome de fluor ou un atome de brome ;
  . un groupement méthoxy ou éthoxy ;

. un groupement aldéhyde ;
. un groupement OH ;
. un groupement CN ;
. un groupement acétoxy ;
. un groupement ester $-COOR_5$, $R_5$ représentant un groupement méthyle ou éthyle ;
. un groupement acyle ayant 2 à 4 atomes de carbone ;

- $R_1$ représente un atome d'hydrogène, un groupement méthyle, éthyle, propyle, isopropyle ou tertiobutyle ou a les significations indiquées pour $R_2$ ;
- $R_3$ et $R_4$ représentent :
  . l'un un atome d'hydrogène ;
  . l'autre un atome d'hydrogène ou un groupement tel que ceux représentés par $R_2$.

**9.** Procédé selon l'une des revendications 1 à 8, caractérisé en ce qu'il est appliqué aux composés phénoliques choisis parmi le chloro-4 phénol, le dichloro-2,4 phénol, le dichloro-3,4 phénol, le chloro-2, fluoro-4 phénol, le chloro-4 fluoro-2 phénol, le bromo-2 chloro-4 phénol, le bromo-4 chloro-2 phénol, le chloro-2 méthoxy-4 phénol, le chloro-4 méthoxy-2 phénol, le chloro-4 méthyl-2 phénol, le méthoxy-4 phénol, l'éthoxy-4 phénol, l'hydroxy-4 acétophénone, l'hydroxy-4 benzonitrile, le chloro-3 hydroxy-4 benzaldéhyde, le chloro-5 hydroxy-2 benzaldéhyde.

**10.** Procédé selon l'une des revendications 1 à 9, caractérisé en ce qu'il est appliqué à un mélange de dichloro-2,4 phénol et de dichloro-2,6 phénol.

**11.** Procédé selon l'une des revendications 1 à 10, caractérisé en ce qu'il est appliqué à un mélange industriel contenant du dichloro-2,4 phénol, du dichloro-2,6 phénol et du trichloro-2,4,6 phénol.

**12.** Procédé selon l'une des revendications 1 à 11, caractérisé en ce qu'il est mis en oeuvre à une température inférieure ou égale à 150 °C et de préférence entre le point de fusion du mélange réactionnel et 120 °C.

## Claims

**1.** Process for the selective chlorination in the ortho position relative to the hydroxyl functional group of phenolic compounds of general formula (I):

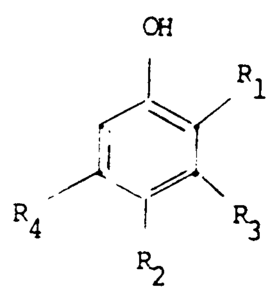

(I)

in which:

- $R_2$ represents:
  a halogen atom;
  an alkyl group containing 1 to 4 carbon atoms;
  an alkoxy group containing 1 to 4 carbon atoms;
  an acyloxy group containing 2 to 4 carbon atoms;
  an acyl group containing 2 to 4 carbon atoms;
  a carboxylic acid group;
  an ester group $-COOR_5$, $R_5$ representing a straight-chain or branched alkyl radical containing from 1 to 4 carbon atoms;
  a nitrile group;
  an OH group;
  a -CHO group;
- $R_1$ represents a hydrogen atom or has one of the meanings given for $R_2$;

- $R_3$ and $R_4$, which may be identical or different, represent a hydrogen atom or an atom or group such as those represented by $R_2$;

with gaseous chlorine, characterised in that the reaction is carried out in a molten medium and in the presence of an effective amount of a primary, secondary or tertiary amine.

**2.** Process according to Claim 1, characterised in that the amines used are more particularly the amines of general formula (II)

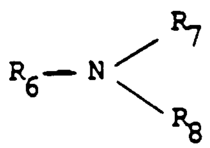

(II)

in which:
- $R_6$, $R_7$ and $R_8$, which may be identical or different, represent:
  a straight-chain alkyl radical containing from 1 to 12 carbon atoms, a secondary alkyl radical containing 3 to 12 carbon atoms or a tertiary alkyl radical containing 4 to 12 carbon atoms, it being possible for these alkyl radicals to contain one or two ether functional groups -O- or hydroxyl, amine, carboxylic acid, carboxylic acid ester, amide or imine functional groups;
  a phenyl radical, a cyclohexyl radical, a cycloheptyl radical or a cyclopentyl radical;
  a phenylalkyl, cyclohexylalkyl, cycloheptylalkyl or cyclopentylalkyl radical in which the alkyl portion contains 1 to 4 carbon atoms;
- one or two of the symbols $R_6$, $R_7$ and $R_8$ may represent a hydrogen atom;
- $R_6$ may represent an $NH_2$ group;
- $R_7$ and $R_8$ together, and with the nitrogen atom, form a saturated heterocyclic ring or a heterocyclic ring containing one or more double bonds, if appropriate substituted with one or more alkyl groups containing 1 to 4 carbon atoms;
- $R_7$ and $R_8$ or $R_6$, $R_7$ and $R_8$, together with the nitrogen atom and with one or more nitrogen and/or oxygen atoms, form a saturated or unsaturated heterocyclic ring, if appropriate substituted with one or more alkyl groups containing 1 to 4 carbon atoms;
- $R_6$, $R_7$ and $R_8$ together, and with the nitrogen atom, form an unsaturated heterocyclic ring, if appropriate substituted with one or two methyl or ethyl groups;
- $R_7$ and $R_8$ or $R_6$, $R_7$ and $R_8$, together with the nitrogen atom and, if appropriate, with one or more nitrogen and/or oxygen atoms, form a saturated or unsaturated polycyclic compound, if appropriate substituted with one or more alkyl groups containing 1 to 4 carbon atoms.

**3.** Process according to one of Claims 1 and 2, characterised in that the quantity of amine used represents, by weight relative to the weight of the reaction mixture, from 0.005% to 5%, and preferably from 0.01% to 2.0%.

**4.** Process according to one of Claims 1 to 3, characterised in that primary or secondary amines of general formula (III) are used:

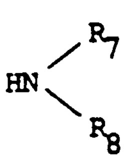

(III)

in which:
- $R_7$ or $R_8$ represents a hydrogen atom;
- $R_7$ and $R_8$, which may be identical or different, represent:
  a straight-chain alkyl radical containing 1 to 10 carbon atoms;
  a secondary alkyl radical containing 3 to 10 carbon atoms;
  a tertiary alkyl radical containing 4 to 10 carbon atoms;

a cyclohexyl or cyclopentyl radical;
a phenyl radical;
a benzyl or phenethyl radical;

- $R_7$ and $R_8$, together with the nitrogen atom and with one other nitrogen and/or oxygen atom, form a saturated heterocyclic ring or a heterocyclic ring containing one or more unsaturations;
- $R_7$ and/or $R_8$ contain one or more amine, hydroxyl or carboxylic acid ester functional groups.

**5.** Process according to one of Claims 1 to 4, characterised in that secondary amines of general formula (III) are used:

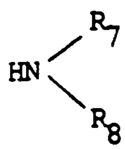

(III)

in which at least one of the symbols $R_7$ and $R_8$, and preferably both symbols $R_7$ and $R_8$ represent:
a secondary alkyl radical containing from 3 to 10 carbon atoms such as isopropyl, 2-butyl, 2-pentyl, 3-pentyl, 2-hexyl, 3-hexyl, 2-heptyl, 3-heptyl, 4-heptyl, 2-octyl, 3-octyl, 4-octyl, 2-nonyl, 3-nonyl, 4-nonyl, 5-nonyl, 2-decyl, 3-decyl, 4-decyl and 5-decyl;
a cyclohexyl or cyclopentyl radical;
or secondary amines of general formula (III) are used in which $R_7$ and $R_8$ form with the nitrogen atom a heterocyclic ring containing, if appropriate, another nitrogen atom or an oxygen atom.

**6.** Process according to one of Claims 1 to 5, characterised in that diisopropylamine, diisobutylamine, dicyclohexylamine, morpholine or imidazole is used as the amine.

**7.** Process according to one of Claims 1 to 6, characterised in that n-propylamine, isopropylamine, n-butylamine, isobutylamine, tert-butylamine, n-pentylamine, 2-methylbutylamine, 3-methylbutylamine, n-hexylamine, 2-methylpentylamine, 3-methylpentylamine, 2-ethylhexylamine, laurylamine, cyclohexylamine, cyclopentylamine, benzylamine, glycine ethyl ether, N-aminomorpholine, N-aminopiperidine, N-aminoethylpyrrolidine or ethanolamine is used as the amine.

**8.** Process according to one of Claims 1 to 7, characterised in that it is applied to phenolic compounds of general formula (I) in which:

- $R_2$ represents:
  a chlorine atom, a fluorine atom or a bromine atom;
  a methoxy or ethoxy group;
  an aldehyde group;
  an OH group;
  a CN group;
  an acetoxy group;
  an ester group $-COOR_5$, $R_5$ representing a methyl or ethyl group;
  an acyl group containing 2 to 4 carbon atoms;
- $R_1$ represents a hydrogen atom, a methyl, ethyl, propyl, isopropyl or tert-butyl group or has the meanings given for $R_2$;
- $R_3$ and $R_4$ represent:
  one a hydrogen atom;
  the other a hydrogen atom or a group such as those represented by $R_2$.

**9.** Process according to one of Claims 1 to 8, characterised in that it is applied to phenolic compounds chosen from 4-chlorophenol, 2,4-dichlorophenol, 3,4-dichlorophenol, 2-chloro-4-fluorophenol, 4-chloro-2-fluorophenol, 2-bromo-4-chlorophenol, 4-bromo-2-chlorophenol, 2-chloro-4-methoxyphenol, 4-chloro-2-methoxyphenol, 4-chloro-2-methylphenol, 4-methoxyphenol, 4-ethoxyphenol, 4-hydroxyacetophenone, 4-hydroxybenzonitrile, 3-chloro-4-hydroxybenzaldehyde and 5-chloro-2-hydroxybenzaldehyde.

10. Process according to one of Claims 1 to 9, characterised in that it is applied to a mixture of 2,4-dichlorophenol and 2,6-dichlorophenol.

11. Process according to one of Claims 1 to 10, characterised in that it is applied to an industrial mixture containing 2,4-dichlorophenol, 2,6-dichlorophenol and 2,4,6-trichlorophenol.

12. Process according to one of Claims 1 to 11, characterised in that it is carried out at a temperature lower than or equal to 150 ° C, and preferably between the melting point of the reaction mixture and 120 ° C.

**Patentansprüche**

1. Verfahren zur selektiven Chlorierung von Phenolverbindungen der allgemeinen Formel (I) in Orthostellung der Hydroxylfunktion mit gasförmigem Chlor:

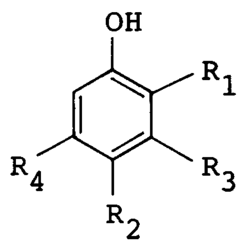

(I)

in der:

- $R_2$
  . ein Halogenatom:
  . eine Alkylgruppe mit 1 bis 4 Kohlenstoffatomen;
  . eine Alkoxygruppe mit 1 bis 4 Kohlenstoffatomen;
  . eine Acyloxygruppe mit 2 bis 4 Kohlenstoffatomen;
  . eine Acylgruppe mit 2 bis 4 Kohlenstoffatomen;
  . eine Carbonsäuregruppe;
  . eine Estergruppe-$COOR_5$, wobei $R_5$ einen geradkettigen oder verzweigten Alkylrest mit 1 bis 4 Kohlenstoffatomen bedeutet;
  . eine Nitrilgruppe;
  . eine OH Gruppe;
  . eine -CHO Gruppe bedeutet;
- $R_1$ ein Wasserstoffatom bedeutet oder eine der für $R_2$ angegebenen Bedeutungen besitzt;
- $R_3$ und $R_4$, gleich oder verschieden, ein Wasserstoffatom oder ein Atom oder eine Gruppierung, wie die durch $R_2$ dargestellte, bedeuten;

dadurch gekennzeichnet, daß man in der Schmelze und in Gegenwart einer wirksamen Menge eines primären, sekundären oder tertiären Amins arbeitet.

2. Verfahren nach Anspruch 1, dadurch gekennzeichnet, daß die verwendeten Amine insbesondere Amine der allgemeinen Formel (II) sind

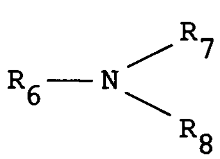

(II)

in der:

- $R_6$, $R_7$ und $R_8$, gleich oder verschieden,

. einen geradkettigen Alkylrest mit 1 bis 12 Kohlenstoffatomen, einen sekundären Alkylrest mit 3 bis 12 Kohlenstoffatomen oder einen tertiären Alkylrest mit 4 bis 12 Kohlenstoffatomen, wobei diese Alkylreste eine oder zwei Etherfunktionen -O- oder Hydroxyl-, Amin-, Carbonsäure-, Carbonsäureester-, Amid- oder Imingruppen enthalten können;
. einen Phenylrest, einen Cyclohexylrest, einen Cycloheptyl- oder Cyclopentylrest;
. einen Phenylalkyl-, Cyclohexylalkyl-, Cycloheptylalkyl- oder Cyclopentylalkylrest, wobei der Alkylteil 1 bis 4 Kohlenstoffatome trägt, bedeuten;

- ein oder zwei der Symbole $R_6$, $R_7$ und $R_8$ ein Wasserstoffatom bedeuten können;
- $R_6$ eine $NH_2$ Gruppe bedeuten kann;
- $R_7$ und $R_8$ gemeinsam und mit dem Stickstoffatom einen gesättigten oder eine oder mehrere Doppelbindungen enthaltenden, gegebenenfalls durch eine oder mehrere Alkylgruppen mit 1 bis 4 Kohlenstoffatomen substituierten Heterozyklus bilden;
- $R_7$ und $R_8$ oder $R_6$, $R_7$ und $R_8$ gemeinsam mit dem Stickstoffatom und mit einem oder mehreren Stickstoff- und/oder Sauerstoffatomen einen gesättigten oder ungesättigten, gegebenenfalls durch eine oder mehrere Alkylgruppen mit 1 bis 4 Kohlenstoffatomen substituierten Heterozyklus bilden;
- $R_6$, $R_7$ und $R_8$ gemeinsam und mit dem Stickstoffatom einen ungesättigten, gegebenenfalls durch eine oder zwei Methyl- oder Ethylgruppen substituierten Heterozyklus bilden;
- $R_7$ und $R_8$ oder $R_6$, $R_7$ und $R_8$ gemeinsam mit dem Stickstoffatom und gegebenenfalls mit einem oder mehreren Stickstoff- und/oder Sauerstoffatomen eine gesättigte oder ungesättigte, gegebenenfalls durch eine oder mehrere Alkylgruppen mit 1 bis 4 Kohlenstoffatomen substituierte polyzyklische Verbindung bilden.

**3.** Verfahren nach einem der Ansprüche 1 und 2, dadurch gekennzeichnet, daß die Menge des verwendeten Amins 0,005 bis 5 und vorzugsweise 0,01 bis 2,0 Gewichtsprozent bezogen auf das Gewicht der Reaktionsmischung beträgt.

**4.** Verfahren nach einem der Ansprüche 1 bis 3, dadurch gekennzeichnet, daß man primäre oder sekundäre Amine der allgemeinen Formel (III):

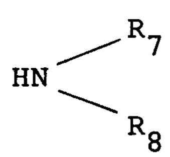

(III)

verwendet, in der:

- $R_7$ oder $R_8$ ein Wasserstoffatom bedeutet;
- $R_7$ und $R_8$, gleich oder verschieden,
  . einen geradkettigen Alkylrest mit 1 bis 10 Kohlenstoffatomen;
  . einen sekundären Alkylrest mit 3 bis 10 Kohlenstoffatomen;
  . einen tertiären Alkylrest mit 4 bis 10 Kohlenstoffatomen;
  . einen Cyclohexyl- oder Cyclopentylrest;
  . einen Phenylrest;
  . einen Benzyl- oder Phenethylrest bedeuten.
- $R_7$ und $R_8$ gemeinsam mit dem Stickstoffatom und mit einem anderen Stickstoff- und/oder Sauerstoffatom einen gesättigten oder eine oder mehrere Unsättigungen aufweisenden Heterozyklus bilden;
- $R_7$ und/oder $R_8$ eine oder mehrere Amin-, Hydroxyl- oder Carbonsäureestergruppen tragen.

**5.** Verfahren nach einem der Ansprüche 1 bis 4, dadurch gekennzeichnet, daß man sekundäre Amine der allgemeinen Formel (III):

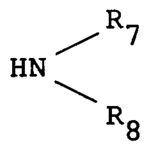

(III)

verwendet, in der wenigstens eines der Symbole $R_7$ und $R_8$ und vorzugsweise die beiden Symbole $R_7$ und $R_8$ bedeuten:

- . einen sekundären Alkylrest mit 3 bis 10 Kohlenstoffatomen wie Isopropyl, 2-Butyl, 2-Pentyl, 3-Pentyl, 2-Hexyl, 3-Hexyl, 2-Heptyl, 3-Heptyl, 4-Heptyl, 2-Octyl, 3-Octyl, 4-Octyl, 2-Nonyl, 3-Nonyl, 4-Nonyl, 5-Nonyl, 2-Decyl, 3-Decyl, 4-Decyl und 5-Decyl;
- . einen Cyclohexyl- oder Cyclopentylrest; oder sekundäre Amine der allgemeinen Formel (III), in der $R_7$ und $R_8$ mit dem Sticktoffatom einen Heterozyklus bilden, der gegebenenfalls ein anderes Stickstoff- oder Sauerstoffatom enthält.

**6.** Verfahren nach einem der Ansprüche 1 bis 5, dadurch gekennzeichnet, daß man als Amin Diisopropylamin, Diisobutylamin, Dicyclohexylamin, Morpholin oder Imidazol verwendet.

**7.** Verfahren nach einem der Ansprüche 1 bis 6, dadurch gekennzeichnet, daß man als Amin n-Propylamin, Isopropylamin, n-Butylamin, Isobutylamin, tert.-Butylamin, n-Pentylamin, 2-Methylbutylamin, 3-Methylbutylamin, n-Hexylamin, 2-Methylpentylamin, 3-Methylpentylamin, 2-Ethylhexylamin, Laurylamin, Cyclohexylamin, Cyclopentylamin, Benzylamin, Glycinethylester, N-Aminomorpholin, N-Aminopiperidin, N-Aminoethylpyrrolidin oder Ethanolamin verwendet.

**8.** Verfahren nach einem der Ansprüche 1 bis 7, dadurch gekennzeichnet, daß Phenolverbindungen der allgemeinen Formel (I) verwendet werden, in der:

- $R_2$
  - . ein Chloratom, ein Fluoratom oder ein Bromatom;
  - . eine Methoxy- oder Ethoxygruppe;
  - . eine Aldehydgruppe;
  - . eine OH Gruppe;
  - . eine CN Gruppe;
  - . eine Acetoxygruppe;
  - . eine $-COOR_5$ Estergruppe, in der $R_5$ eine Methyl- oder Ethylgruppe bedeutet;
  - . eine Acylgruppe mit 2 bis 4 Kohlenstoffatomen bedeutet;
- $R_1$ ein Wasserstoffatom, eine Methyl-, Ethyl-, Propyl-, Isopropyl- oder tert-Butylgruppe oder eine der für $R_2$ angegebenen Bedeutungen hat;
- $R_3$ und $R_4$
  - . einer ein Wasserstoffatom
  - . der andere ein Wasserstoffatom oder eine Gruppierung, wie sie durch $R_2$ dargestellt wird, bedeuten.

**9.** Verfahren nach einem der Ansprüche 1 bis 8, dadurch gekennzeichnet, daß Phenolverbindungen verwendet werden, die ausgewählt sind, aus 4-Chlorphenol, 2,4-Dichlorphenol, 3,4-Dichlorphenol, 2-Chlor-4-Fluorphenol, 4-Chlor-2-Fluorphenol, 2-Brom-4-Chlorphenol, 4-Brom-2-Chlorphenol, 2-Chlor-4-Methoxyphenol, 4-Chlor-2-Methoxyphenol, 4-Chlor-2-Methylphenol, 4-Methoxyphenol, 4-Ethoxyphenol, 4-Hydroxyacetophenon, 4-Hydroxybenzonitril, 3-Chlor-4-Hydroxybenzaldehyd und 5-Chlor-2-Hydroxybenzaldehyd.

**10.** Verfahren nach einem der Ansprüche 1 bis 9, dadurch gekennzeichnet, daß es auf ein Gemisch von 2,4-Dichlorphenol und 2,6-Dichlorphenol angewendet wird.

**11.** Verfahren nach einem der Ansprüche 1 bis 10, dadurch gekennzeichnet, daß es auf ein industrielles Gemisch, das 2,4-Dichlorphenol, 2,6-Dichlorphenol und 2,4,6-Trichlorphenol enthält, angewendet wird.

**12.** Verfahren nach einem der Ansprüche 1 bis 11, dadurch gekennzeichnet, daß es bei einer Temperatur unter oder gleich 150 ° C und vorzugsweise zwischen dem Schmelzpunkt des Reaktionsgemisches und 120 ° C durchgeführt wird.